# EUROPEAN PATENT APPLICATION

(11) **EP 4 840 963 A1**
(43) Date of publication of application: **18.11.2026**
(21) Application number: 25747443.7
(22) Date of filing: 07.01.2025
(51) Int. Cl.: C12N 15/82, A01H 1/02, A01H 5/02, A01H 6/46

(54) **MALE FERTILITY-RELATED GENE AND USE THEREOF**

(30) Priority: 04.02.2024 CN 202410162602
(71) Applicant: Shandong Laboratory of Advanced Agricultural Sciences in Weifang, Weifang, Shandong 261325 (CN)
(72) Inventor: WANG, Zheng, Weifang, Shandong 261325 (CN); LI, Jian, Weifang, Shandong 261325 (CN); ZHANG, Guoliang, Weifang, Shandong 261325 (CN); LONG, Qiang, Weifang, Shandong 261325 (CN); DENG, Xing Wang, Weifang, Shandong 261325 (CN)
(74) Representative: CAPRI
(86) International application number: PCT/CN2025/070964
(87) International publication number: WO 2025/161867

(57) **Abstract**

Provided are a male fertility-related gene and a use thereof, which belongs to the field of biotechnology. Phenotypic and genetic analysis was performed on the wheat male sterile mutant ms9 identified from an EMS-mutagenized population, and the wheat fertility-related gene *TaMS9* was isolated via MutMap analysis of re-sequencing data in combination with the conventional map-based cloning method. The cloned wheat fertility gene and its male sterile mutant thereof are applied to the next-generation wheat hybrid breeding technology, which have important theoretical and practical significance for the utilization of wheat heterosis.

## Description

### Technical Field

The present disclosure belongs to the field of plant biotechnology, and in particular relates to the cloning of a plant recessive genic sterility gene, the propagation method of a male sterile line thereof, and the application thereof in hybrid breeding. More particularly, the present disclosure relates to the cloning of a recessive genic male sterility gene in *Triticum aestivum* and the promoter thereof, as well as the application thereof in hybrid breeding.

### Background

Wheat is one of the most important grain crops in modern agriculture, serving as a staple food for approximately 35% of the global population. With the development of economy and the growth of population, the demand for wheat yield has been on the rise. However, conventional breeding methods alone struggle to boost wheat yields, and fully exploiting wheat heterosis has emerged as the optimal strategy to comprehensively improve wheat production capacity.

Since wheat is a self-pollinating plant with bisexual flowers, artificial emasculation is impractical for hybrid seed production. Therefore, male sterility is an important agronomic trait for utilizing heterosis to increase crop yield, and the identification of male sterile mutants and the cloning of male fertility genes are the basis for developing hybrid seed production systems. Male sterility in wheat can be divided into two major types: genic male sterility (GMS) and cytoplasmic male sterility (CMS). Based on inheritance patterns, genic male sterile mutants are further classified into dominant and recessive types. Dominant GMS mutants, constrained by their genetic characteristics, are only applicable for conventional breeding approaches such as recurrent selection and backcross breeding, and cannot function as female parents for hybrid seed production. By contrast, recessive GMS mutants possess readily restorable sterility traits, rendering them ideal female parents for hybrid wheat seed production.

As wheat is an allohexaploid species, functional redundancy among the three sub-genomes results in significantly fewer single-gene controlled recessive GMS mutants in wheat compared with other diploid species. To date, only three single-gene controlled recessive genic male fertility loci have been reported in wheat, namely MS1, MS5, and NWMS1 (Klindworth et al., 2002; Li et al., 2019). Among these, the *MS1* and *MS5* genes have been cloned (Tucker et al., 2017; Wang et al., 2017; Pallotta et al., 2019). *MS1* gene is a newly evolved gene in the *Poaceae* and encodes a lipid transfer protein, and *ms1* is the most widely used genic GMS mutant in practical application (Wang et al., 2017; Tucker et al., 2017). The *MS5* gene also encodes a lipid transfer protein different from the MS1 protein (Pallotta et al., 2019).

In order to obtain more stable and reliable recessive GMS mutants and their corresponding genes thereof for application in wheat hybrid breeding, we previously screened a wheat EMS mutant library and successfully identified a large number of male sterile mutants. Among them, *ms9* is a novel recessive GMS mutant, whose sterility is strictly controlled by a single nuclear gene. Phenotypically, *ms9* mutant exhibits a distinct trait of completely empty anthers, with no viable or aborted pollen grains detectable in locules upon microscopic examination. The present disclosure provides genetic analysis, gene cloning and promoter characterization of the *ms9* mutant, and demonstrates the successful application of the *ms9* mutant and its corresponding *TaMS9* gene in a next-generation hybrid wheat breeding system.

### Summary

All references mentioned herein are incorporated by reference.

Unless indicated to the contrary, all technical and scientific terms used herein have the same meaning as commonly understood by one of ordinary skill in the art to which this disclosure belongs. Unless indicated to the contrary, the techniques used or referenced herein are standard techniques well known to those of ordinary skill in the art. The materials, methods, and examples are illustrative only and not limiting.

The present disclosure provides a fertility-related gene *TaMS9*, a nucleotide sequence of the fertility-related gene *TaMS9* is selected from any one of the sequences in the following group:
(a) the nucleotide sequence as shown in SEQ ID NO: 1 or 2;
(b) a nucleotide sequence encoding the amino acid sequence as shown in SEQ ID NO: 3;
(c) a DNA sequence capable of hybridizing with the DNA sequence described in (a) or (b) under a stringent condition; or
(d) a DNA sequence having at least 80% (preferably at least 85%) sequence similarity to any one of the sequences described in (a) - (c) and possessing a fertility restoration function; or
(e) a DNA sequence complementary to any one of the sequences described in (a) - (d).

One skilled in the art would have been aware that the fertility-related gene of the present disclosure also comprises homologous genes: having a nucleotide sequence with high sequence similarity to the nucleotide sequence of *TaMS9* gene; or encoding a protein whose amino acid sequence has high sequence similarity to the amino acid sequence of the protein encoded by the *TaMS9* gene, wherein the homologous gene has the same function of regulating or restoring fertility as the *TaMS9* gene. The homologous gene with fertility-regulating function includes a nucleotide sequence that hybridizes with the nucleotide sequences shown in SEQ ID NO: 1 or 2 under stringent conditions. Or the homologous gene with fertility-regulating function includes a nucleotide sequence encoding an amino acid sequence which has 85% or more similarity to the amino acid sequence as shown in SEQ ID NO: 3. "Stringent conditions" as used herein are well known and include, for example, hybridizing for 12 to 16 hours at 53°C to 60°C in a hybridization solution containing 400 mM NaCl, 40 mM PIPES (pH 6.4), and 1 mM EDTA, followed by washing for 15 to 60 minutes at 62°C to 68°C in a wash solution containing 0.5 x SSC, and 0.1% SDS.

The homologous gene further comprises a DNA sequence having at least 80%, 85%, 90%, 95%, 98%, or 99% sequence similarity to the full-length sequence shown in SEQ ID NO: 1 or SEQ ID NO: 2 and has a fertility regulation function. The homologous gene can be isolated from any plant. The percentage of sequence similarity can be determined by conventional bioinformatic algorithms, including Myers and Miller algorithm, Needleman-Wunsch global alignment, Smith-Waterman local alignment, Pearson and Lipman similarity search, Karlin and Altschul algorithm. This is well known to those skilled in the art.

The present disclosure also provides an expression cassette. The expression cassette contains a DNA sequence of a fertility-related gene disclosed in the present disclosure. The nucleotide sequence of the fertility-related gene is selected from any one of the sequences in the following group:
(a) the nucleotide sequence as shown in SEQ ID NO: 1 or 2.
(b) the nucleotide sequence encoding the amino acid sequence as shown in SEQ ID NO: 3.
(c) a DNA sequence capable of hybridizing with the DNA sequence described in (a) or (b) under stringent conditions; or
(d) a DNA sequence that has at least 80% (preferably at least 85%) sequence similarity with the sequence described in (a) - (c) and possesses a fertility restoration function. or
(e) a DNA sequence complementary to any one of the sequences described in (a) to (d).

In particular, the fertility-related gene in the expression cassette is operably linked to a promoter that drives its expression, including but not limited to, a constitutive expression promoter, an inducible promoter, a tissue-specific expression promoter, or a spatiotemporal specific expression promoter. More specifically, the promoter is a pollen-specific promoter. Preferably, the nucleotide sequence of the pollen-specific promoter is as shown in SEQ ID NO: 12.

The expression cassette of the present disclosure further comprises a pollen inactivation gene, wherein the pollen inactivation gene can interfere with the function or formation of male gametes containing the pollen inactivation gene in a plant. The pollen inactivation gene includes, but is not limited to, a barnase gene, an amylase gene, a DAM methylase gene, and the like. More specifically, the pollen inactivation gene is the maize alpha amylase gene, and the nucleotide sequence thereof is as shown in SEQ ID NO: 15.

The expression cassette of the present disclosure further comprises a selection marker gene, wherein the selection marker gene can be used for selecting a plant, a plant tissue cell or a vector containing the expression cassette. The selection marker gene includes, but is not limited to, a blue-grained gene, an antibiotic resistance gene, a herbicide resistance gene, a fluorescent protein gene, and the like. Specifically, the selection marker gene includes, but is not limited to, a blue-grained gene, a chloramphenicol resistance gene, a hygromycin resistance gene, a streptomycin resistance gene, a spectinomycin resistance gene, a sulfonamide resistance gene, a glyphosate resistance gene, a phosphinothricin resistance gene, a bar gene, a red fluorescent gene *DsRED*, an *mCherry* gene, a cyan fluorescent protein gene, a yellow fluorescent protein gene, a luciferase gene, a green fluorescent protein gene, and the like.

Also disclosed is a method for regulating fertility of a plant. The method restores male fertility of a wheat *ms9* mutant by introducing a fertility-related gene into the wheat *ms9* mutant, wherein the nucleotide sequence of the fertility-related gene is selected from any one of the sequences in the following group:
(a) the nucleotide sequence as shown in SEQ ID NO: 1 or 2;
(b) a nucleotide sequence encoding the amino acid sequence as shown in SEQ ID NO: 3;
(c) a DNA sequence capable of hybridizing with the DNA sequence described in (a) or (b) under a stringent condition; or
(d) a DNA sequence having at least 80% (preferably at least 85%) sequence similarity to any one of the sequences described in (a) - (c) and possessing a fertility restoration function; or
(e) a DNA sequence complementary to any one of the sequences described in (a) - (d).

Also disclosed is a method for maintaining a male sterile line. The method comprises: using a wheat *ms9* mutant plant as a genetic transformation recipient, and introducing three closely linked target genes into the sterile mutant recipient. The three closely linked target genes are a fertility-related gene *TaMS9,* a pollen inactivation gene, and a selection marker gene, respectively. The fertility-related gene *TaMS9* is capable of restoring fertility to the sterile transformation recipient. The pollen inactivation gene inactivates pollen harboring the transformed exogenous gene, i.e., eliminating its fertilization ability. The selection marker gene enables sorting of transgenic versus non-transgenic seeds or tissues; the sorted non-transgenic seeds are used as a male sterile-line to produce hybrid seeds, and the transgenic seeds are used as the maintainer line to continuously and stably produce the sterile line.

In the described method for maintaining a male sterile line, the pollen inactivation gene comprises, but is not limited to, a barnase gene, an amylase gene, a DAM methylase gene, etc. More specifically, the pollen inactivation gene is the maize alpha amylase gene *Zm-AA*, and the nucleotide sequence thereof is as shown in SEQ ID NO: 15. The pollen inactivation gene is linked to a promoter that preferentially drives expression in male gametes. More specifically, such male gamete-preferential promoters include, but are not limited to, the *PG47* promoter, the *Zm13* promoter, and the like. The selection marker gene can be used to select out plants or vectors containing the expression cassette. Such selection marker genes include, but are not limited to, a blue-grained gene, an antibiotic resistance gene, an herbicide resistance gene, a fluorescent protein gene, and the like. Specifically, the selection marker genes include, but are not limited to, a blue-grained gene, a chloramphenicol resistance gene, a hygromycin resistance gene, a streptomycin resistance gene, a spectinomycin resistance gene, a sulfonamide resistance gene, a glyphosate resistance gene, a phosphinothricin resistance gene, a bar gene, a red fluorescent gene *DsRED*, an *mCherry* gene, a cyan fluorescent protein gene, a yellow fluorescent protein gene, a luciferase gene, a green fluorescent protein gene, and the like.

More specifically, also disclosed is a method for propagating a male sterile line. The method comprises the following steps:
(a) introducing the following vector into the wheat *ms9* male sterile line to obtain a maintainer line harboring the vector, the vector comprises:
   a fertility-related gene *TaMS9*, the fertility-related gene *TaMS9* is capable of restoring the male fertility of the wheat *ms9* mutation; and
   a pollen inactivation gene, when expressed in a plant, disturbs the function or formation of a male gamete harboring the pollen inactivation gene, so the fertile male gamete generated in the plant dose not comprise the vector; and
   a selection marker gene, the selection marker gene is used for sorting transgenic seeds or tissues from non-transgenic seeds or tissues; and
(b) the maintainer line plant harboring the above vector is self-pollinated, thereby producing both seeds of the wheat *ms9* male sterile line not comprising the vector and seeds of the maintainer line comprising the vector are produced, or plants of the wheat *ms9* male sterile line are pollinated with pollen grains of the maintainer line plant, thereby propagating seeds of the *ms9* male sterile line or the allelic sterile line thereof.

In the described method for propagating a male sterile line, the pollen inactivation gene comprises, but is not limited to, a barnase gene, an amylase gene, a DAM methylase gene, etc. More specifically, the pollen inactivation gene is the maize alpha amylase gene *Zm-AA,* and the nucleotide sequence thereof is as shown in SEQ ID NO: 15. The pollen inactivation gene is linked to a promoter that preferentially drives expression in male gametes. More specifically, such male gamete-preferential promoters include, but are not limited to, the *PG47* promoter, the *Zm13* promoter, and the like. The selection marker gene can be used to select out plants or vectors containing the expression cassette. Such selection marker genes include, but are not limited to, a blue-grained gene, an antibiotic resistance gene, an herbicide resistance gene, a fluorescent protein gene, and the like. Specifically, the selection marker genes include, but are not limited to, a blue-grained gene, a chloramphenicol resistance gene, a hygromycin resistance gene, a streptomycin resistance gene, a spectinomycin resistance gene, a sulfonamide resistance gene, a glyphosate resistance gene, a phosphinothricin resistance gene, a bar gene, a red fluorescent gene *DsRED,* an *mCherry* gene, a cyan fluorescent protein gene, a yellow fluorescent protein gene, a luciferase gene, a green fluorescent protein gene, and the like.

Also disclosed is a method for producing a maintainer line. The method comprises the following steps:
(a) introducing the following vector into the wheat *ms9* male sterile line to obtain a maintainer line for the wheat *ms9* genic male sterile line or the allelic sterile line thereof; the vector comprises:
   a fertility-related gene *TaMS9,* the fertility-related gene *TaMS9* is capable of restoring the male fertility of the wheat *ms9* genic male sterile line or the allelic sterile line thereof; and
   a pollen inactivation gene, when expressed in a plant, disturbs the function or formation of a male gamete harboring the pollen inactivation gene, so the fertile male gametes generated in the plant do not harbor the vector; and
   a selection marker gene, the selection marker gene is used for sorting transgenic seeds from non-transgenic seeds.

In the above method for producing the maintainer line, the pollen inactivation gene comprises, but is not limited to, a barnase gene, an amylase gene, a DAM methylase gene, etc. More specifically, the pollen inactivation gene is the maize alpha amylase gene *Zm-AA,* and the nucleotide sequence thereof is as shown in SEQ ID NO: 15. The pollen inactivation gene is linked to a promoter that preferentially drives expression in male gametes. More specifically, such male gamete-preferential promoters include, but are not limited to, the *PG47* promoter, *Zm13* promoter, and the like. The selection marker gene can be used to select out plants or vectors containing the expression cassette. Such selection marker genes include, but are not limited to, a blue-grained gene, an antibiotic resistance gene, an herbicide resistance gene, a fluorescent protein gene, and the like. Specifically, the selection marker genes include, but are not limited to, a blue-grained gene, a chloramphenicol resistance gene, a hygromycin resistance gene, a streptomycin resistance gene, a spectinomycin resistance gene, a sulfonamide resistance gene, a glyphosate resistance gene, a phosphinothricin resistance gene, a bar gene, a red fluorescent gene *DsRED,* an *mCherry* gene, a cyan fluorescent protein gene, a yellow fluorescent protein gene, a luciferase gene, a green fluorescent protein gene, and the like.

Also disclosed is a method for propagating a maintainer line, comprising the steps of:
(a) introducing the following vector into the wheat *ms9* genic male sterile line or its allelic sterile line thereof to obtain a maintainer line for the wheat *ms9* genic male sterile line or its allelic sterile line thereof; the vector comprises:
   a fertility-related gene *TaMS9,* the fertility-related gene *TaMS9* is capable of restoring the male fertility of the wheat *ms9* genic male sterile line or the allelic sterile line thereof; and
   a pollen inactivation gene, when expressed in a plant, disturbs the function or formation of a male gamete harboring the pollen inactivation gene, so the fertile male gametes generated in the plant do not harbor the vector; and
   a selection marker gene, the selection marker gene is used for sorting transgenic seeds from non-transgenic seeds; and
(b) the maintainer line harboring the above vector is self-pollinated, thereby propagating seeds of the wheat *ms9* genic male sterile line without the vector or the allelic sterile line thereof and the maintainer line harboring the vector in a ratio of 1:1.

Also disclosed is a method for producing seeds. The method comprises:
(a) introducing the following vector into the wheat *ms9* genic male sterile line or the allelic sterile line thereof to obtain a maintainer line for a wheat *ms9* genic male sterile line or an allelic sterile line thereof; the vector comprises:
   a fertility-related gene *TaMS9,* the fertility-related gene *TaMS9* is capable of restoring the male fertility of the wheat *ms9* genic male sterile line or the allelic sterile line thereof; and
   a pollen inactivation gene, when expressed in a plant, disturbs the function or formation of a male gamete comprising the pollen inactivation gene, so the fertile male gametes generated in the plant do not harbor the vector;
(b) self-pollinating the maintainer line harboring the vector; and
(c) obtaining both seeds of the maintainer line harboring the vector and seeds of the wheat *ms9* genic male sterile line and the allelic sterile line thereof without the vector.

The present disclosure relates to the methods for propagating or maintaining a male sterile line, producing or propagating the maintainer line, producing seeds, and the like, wherein step (a) may also be introducing the vector containing the fertility-related gene *TaMS9,* a pollen inactivation gene and a selection marker gene into a common wheat plant, thereby obtaining a transgenic plant harboring the vector, crossing the obtained transgenic plant with a wheat *ms9* genic male sterile line or its allelic sterility line thereof, obtaining a maintainer plant harboring the vector in the background of the wheat *ms9* genic male sterile line or the allelic sterile line thereof via directional breeding.

The present disclosure relates to the methods for propagating or maintaining a male sterile line, producing or propagating the maintainer line, producing seeds, and the like. The nucleotide sequence of the fertility-related gene is selected from any one of the following sequences:
(a) the nucleotide sequence as shown in SEQ ID NO: 1 or 2;
(b) a nucleotide sequence encoding the amino acid sequence as shown in SEQ ID NO: 3;
(c) a DNA sequence capable of hybridizing with the DNA sequence described in (a) or (b) under a stringent condition; or
(d) a DNA sequence having at least 80% (preferably at least 85%) sequence similarity to any one of the sequences described in (a) - (c) and possessing a fertility restoration function; or
(e) a DNA sequence complementary to any one of the sequences described in (a) - (d).

The fertility-related gene *TaMS9* is also operably linked to a pollen-specific promoter that drives expression of the *TaMS9* gene in plant pollen. The pollen-specific promoter is selected from the group consisting of the promoters of fertility regulatory genes MS26, NP1, MSP1, PAIR1, PAIR2, ZEP1, MELL, PSS1, TDR, UDT1, GAMYB4, PTC1, API5, WDA1, CYP704B2, MS26, MS22, DPW, MADS3, OSC6, RIP1, CSA, AID1, 5126 or Ms45. More specifically, the nucleotide sequence of the pollen-specific promoter is as shown in SEQ ID NO: 10. The fertility-related gene may also be operably linked to a terminator which may be a terminator from any previously disclosed genes, and particularly the nucleotide sequence of one the terminator is as shown in SEQ ID NO: 13. In the described method for propagating or maintaining a male sterile line, the method for producing or propagating a maintainer line, and the method for producing seeds, etc. of the present disclosure, the pollen inactivation gene comprises, but is not limited to, a barnase gene, an amylase gene, a DAM methylase gene, etc. More specifically, the pollen inactivation gene is the maize alpha amylase gene *Zm-AA*, and the nucleotide sequence thereof is as shown in SEQ ID NO: 15. The pollen inactivation gene is linked to a promoter that preferentially drives expression in male gametes. More specifically, such male gamete-preferential promoters include, but are not limited to, the *PG47* promoter, the *Zm13* promoter, and the like.

The foregoing methods of the present disclosure for propagating or maintaining a male sterile line, producing or propagating a maintainer line, or producing seeds, wherein the selection marker gene includes, but is not limited to, a blue-grained gene, an antibiotic resistance gene, a herbicide resistance gene, or a fluorescent gene. Specifically, the selection marker genes include, but are not limited to: a blue-grained gene, a chloramphenicol resistance gene, a hygromycin resistance gene, a streptomycin resistance gene, a spectinomycin resistance gene, a sulfonamide resistance gene, a glyphosate resistance gene, a phosphinothricin resistance gene, a bar gene, a red fluorescent gene *DsRED*, a mCherry gene, a cyan fluorescent protein gene, a yellow fluorescent protein gene, a luciferase gene, a green fluorescent protein gene, and the like.

The present disclosure also provides a pollen-specific promoter, the nucleotide sequence of pollen-specific promoter is as shown in SEQ ID NO: 12. The SEQ ID NO: 12 is inserted into a vector and connected with a reporter gene GUS. The vector was then introduced into rice and wheat. GUS expression activity and expression pattern in transgenic plants were detected and analyzed through GUS staining analysis in the roots, stems, leaves and flowers of the transgenic plant. It was found that the promoter provided in the present disclosure drives expression of the GUS gene specifically in plant pollen. The SEQ ID NO: 12 provided by the present disclosure is a promoter that expresses specifically in pollen.

The plant pollen-specific promoter provided by the present disclosure contains the nucleotide sequence shown as SEQ ID NO: 12 in the Sequence Listing, or contains nucleotide sequences having more than 90% similarity with the nucleotide sequences listed in SEQ ID NO: 12, or contains 500 or more consecutive nucleotide fragments from the nucleotide sequence of SEQ ID NO: 12; wherein the promoter is capable of driving expression of an operably linked nucleotide sequence in plant pollen. An expression vector, a transgenic cell line and a host bacterium harboring any of the sequences described above are all within the scope of protection of the present disclosure. Primer pairs that are used to amplify any nucleotide fragment of the promoter having the nucleotide sequence shown as SEQ ID NO: 12 are also within the scope of the present disclosure.

A "promoter" as used herein refers to a DNA regulatory region which typically comprises a TATA box capable of directing RNA polymerase II to initiate RNA synthesis at the appropriate transcription initiation site for a particular coding sequence. The promoter may also comprise other recognition sequences which are normally located upstream or at the 5' end of the TATA box, commonly referred to as upstream promoter elements, and which function to regulate transcription efficiency. It will be appreciated by those of skill in the art that while the present disclosure provides identified promoter nucleotide sequences, it is also within the scope of this disclosure to isolate and identify other regulatory elements located upstream of the TATA box of the particular promoter region disclosed herein. Thus, the promoter regions disclosed herein are generally further defined as sequences comprising upstream regulatory elements (e.g., enhancers, and the like) that direct the tissue-specific and temporal-specific expression of a coding sequence. In the same manner, the promoter elements that enable expression of a coding sequence in the target tissue (e.g., male tissue) can be identified, isolated, and may be combined with other core promoters to verify male tissue-preferred expression. The core promoter is the minimal sequence required to initiate transcription, e.g., the sequence known as the TATA box, which is typically conserved among promoters of protein-coding genes. Thus, alternatively, the upstream promoter of *TaMS9* gene can be used in association with its native core promoter or the core promoter from other sources.

The core promoter may be any of the various known core promoters, for example the cauliflower mosaic virus 35S or 19S promoter (US Patent No. 5,352,605), the ubiquitin promoter (US Patent No. 5,510,474), the IN2 core promoter (US Patent No. 5,364,780) or the figwort mosaic virus promoter.

The function of the gene promoter can be analyzed by the following method: operably linking a promoter sequence to a reporter gene to construct a transformable vector, introducing the vector into a plant, generating transgenic progeny, and characterizing the expression pattern of the reporter gene by observing its activity across various tissues and organs of the transgenic plant; alternatively, the promoter sequence may be subcloned into an expression vector for transient expression assay to detect the function of the promoter or the regulatory region thereof.

The choice of an appropriate expression vector to test the function of a promoter or regulatory region will depend on the host and the method of introducing the expression vector into the host, such methods being well known to those of ordinary skill in the art. For eukaryotes, the sequence in the vector includes regions that regulate transcription initiation and control processing. These regions are operably linked to a reporter gene comprising *YFP*, *UidA*, *GUS* gene, or luciferase. Expression vectors containing putative regulatory regions from genomic fragments can be introduced into intact tissues, such as pollen at the developmental stage, or into callus for functional validation.

In addition, the promoters of the present disclosure can be operably linked to nucleotide sequences other than the *TaMS9* gene to express other heterologous nucleotide sequences. The promoter nucleotide sequences, and fragments and variants thereof, of the present disclosure can be assembled with heterologous nucleotide sequences into a single expression cassette for expression in a plant of interest, more particularly in the male organs of the plant. The expression cassette has suitable restriction sites for insertion of the promoter and the heterologous nucleotide sequence. These expression cassettes can be used for genetic manipulation of any plant to obtain the desired corresponding phenotype.

The pollen-specific promoter disclosed in the present disclosure can be used to drive the expression of the following heterologous nucleotide sequences, thereby conferring a male sterile phenotype to the transformed plant; the heterologous nucleotide sequences may encode an enzyme that promotes carbohydrate degradation, a modified enzyme, an amylase, a debranching enzyme or a pectinase, more particularly, the sequences may be the barnase gene, the maize alpha amylase gene, the auxin gene, the *rotB* gene, the cytotoxin gene, the diphtheria toxin, the DAM methylase, or a dominant male sterility gene.

In certain embodiments, the nucleotide sequence referred to in the present disclosure is operably linked downstream of the promoter of the present disclosure, wherein the "nucleotide sequence" may be a structural gene, a regulatory gene, an antisense gene targeting to a structural gene, an antisense gene targeting to a regulatory gene, or a small RNA that interferes with the expression of an endogenous gene.

The present disclosure also provides a transcription terminator sequence. The nucleotide sequence of the transcription terminator is as shown in SEQ ID NO: 13, and has the function of terminating the gene transcription.

The present disclosure also provides an expression cassette, a vector, or an engineering strain. The expression cassette, the vector or the engineering strain comprises a pollen-specific promoter SEQ ID NO: 12 provided by the present disclosure. Specifically, the nucleotide sequence of the fertility-related gene *TaMS9* provided by the present disclosure can be constructed downstream of the promoter SEQ ID NO: 12 provided by the present disclosure to drive expression of the fertility gene in a transformation recipient plant.

The pollen-specific promoter provided in the present disclosure can be used for specific expression of an exogenous gene in pollen, thereby avoiding the negative effect caused by constitutive expression of the exogenous gene in other plant tissues, and can also be used for functional analysis and identification of genes related to plant pollen growth and development; can be used for the creation of male sterile lines and maintainer lines; furthermore, the promoter can also be applied in pollen sterilization experiments to avoid the bio-safety risk resulting from transgene flow or pollen escape, and therefore has great significance for the creation of plant male sterile lines and maintainer lines.

The nucleotide sequence, promoter sequence or expression cassette of the *TaMS9* gene provided in the present disclosure can be inserted into a vector, plasmid, yeast artificial chromosome, bacterial artificial chromosome, or any other vector suitable for transformation into a host cell. Preferred host cells are bacterial cells, especially bacterial cells for cloning or storing polynucleotides, or for transforming plant cells, such as *Escherichia coli*, *Agrobacterium tumefaciens*, and *Agrobacterium rhizogenes*. When the host cell is a plant cell, the expression cassette or vector can be inserted into the genome of the transformed plant cell. The insertion may be site-specific or random.

The introduction or transformation of a nucleotide sequence, a vector or an expression cassette into a plant in the present disclosure refers to delivering the nucleotide sequence, vector or expression cassette into a recipient cell or plant by conventional transgenic methods. Any transgenic method known to those skilled in the art of plant biotechnology can be used to introduce a recombinant expression vector into a plant cell to produce the transgenic plant of the disclosure. Transformation methods can include both direct and indirect transformation methods. Suitable direct methods include polyethylene glycol-induced DNA uptake, liposome-mediated transformation, biolistic introduction, electroporation, and microinjection. The transformation methods also include *Agrobacterium*-mediated plant transformation methods and the like.

Compared with the prior art, the present disclosure has the following beneficial effects: the present disclosure provides a fertility-related *TaMS9* gene and the promoter thereof, and a method in which *TaMS9* gene is used for the propagation and maintaining of *ms9* male sterile line. The fertility-related gene and the method for maintaining fertility of a genic male sterile line and propagating a sterile line provided by the present disclosure have significant production promotion and application value for hybrid breeding production of crops. The fertility gene and the male sterile line generated by mutation of the gene provided by the present disclosure provide genetic resources for wheat hybrid breeding, and also provide necessary elements for constructing a third-generation hybrid breeding system. The male sterile line generated by mutation of the gene is used for producing hybrid seeds, which is of great significance for breaking through and improving existing "three-line" and "two-line" hybrid technologies.

### References

1. Klindworth D L, Williams N D, Maan S. Chromosomal location of genetic male sterility genes in four mutants of hexaploid wheat. Crop Sci, 2002, 42: 1447-1450.
2. Li J, Zhang J, Li H, et al. The Major Factors Causing the Microspore Abortion of Genic Male Sterile Mutant NWMS1 in Wheat (Triticum aestivum L.). Int J Mol Sci, 2019, 20:6252.
3. Tucker E J, Baumann U, Kouidri A, et al. Molecular identification of the wheat male fertility gene MS1 and its prospects for hybrid breeding. Nat Commun, 2017, 8:869.
4. Wang Z, Li J, Chen S X, et al. Poaceae-specific MS1 encodes a phospholipid-binding protein for male fertility in bread wheat. Proc Nat1 Acad Sci USA, 2017, 114: 12614-12619.
5. Pallotta M A, Warner P, Kouidri A, et al. Wheat ms5 male-sterility is induced by recessive homoeologous A and D genome non-specific lipid transfer proteins. Plant J, 2019, 99:673-685.

### Brief Description of the Drawings

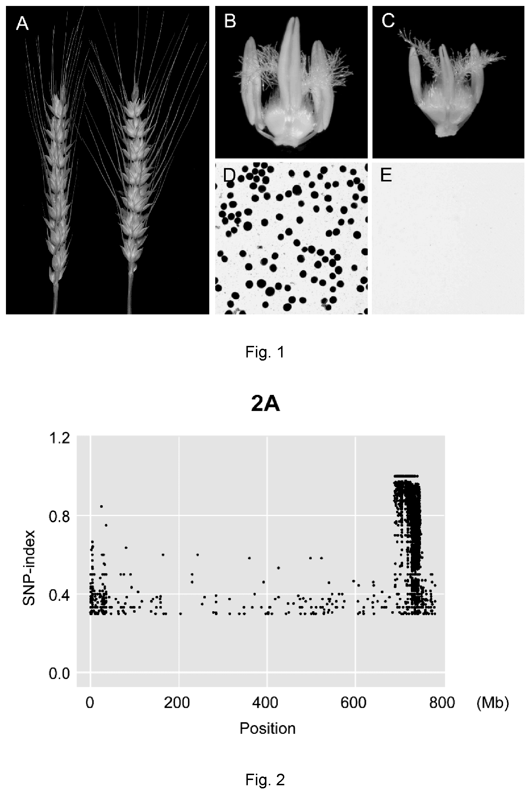

Fig. 1 shows the phenotype of the wheat *ms9* mutant. Panel A displays the spikes at anthesis from the parental line Ningchun 4 (left) and the *ms9* mutant (right). Panels B and C show the anther development in Ningchun 4 and the *ms9* mutant *ms9*, respectively. Panels D and E present the mature pollen grains stained with I₂-KI from Ningchun 4 and the *ms9* mutant, respectively.
Fig. 2 shows the SNP index plot for 2A chromosome of the *ms9* mutant, analyzed using MutMap after re-sequencing.

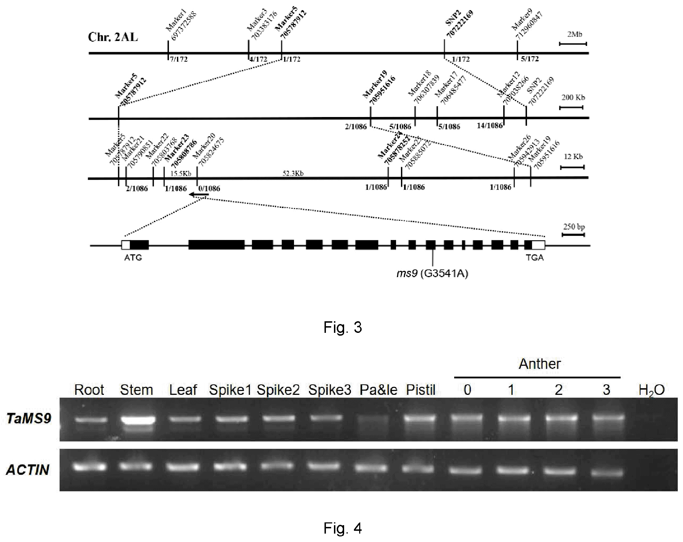

Fig. 3 shows the mapping results of the *TaMS9* gene. The top panel presents the primary mapping results, while the middle two panels show the fine mapping results. The bottom panel displays the gene structure of the candidate gene and the identified mutation sites.
Fig. 4 shows the expression pattern of the *TaMS9* gene in different tissues of Chinese Spring wheat, analyzed by semi-quantitative RT-PCR. The upper panel displays the expression of the *TaMS9* gene in various organs or tissues, while the lower panel shows the expression of the internal control gene *ACTIN.* Spike 1 represents spikes of 1-2 cm in length, Spike 2 represents spikes of 2-3 cm in length, Spike 3 represents spikes of 3-4 cm in length, 0 represents the meiosis stage, 1 represents the uninucleate stage, 2 represents the binucleate stage, 3 represents the trinucleate stage, and "Pa&le" represents a mixed sample of palea and lemma.

### Detailed Description of the Embodiments

### Example1. ms9 is a pollen-free recessive genic male sterile mutant in wheat

In this disclosure, large-scale phenotypic screening was conducted among an EMS-mutagenized population in spring wheat cultivar Ningchun4, resulting in the identification of multiple male sterile mutants. A novel recessive GMS mutant controlled by a single gene was identified through further genetic analysis. Allelic tests confirmed that its locus is non-allelic to all previously reported male sterile loci in wheat, and the locus was named as MS9.

In the F₂ population derived from the cross between *ms9* mutant and its wild type parent Ningchun 4, the segregation ratio of fertile to sterile plants was 126:32. The F₂ populations derived from crosses of *ms9* mutant with wheat cultivars Chinese Spring and Fielder exhibited fertile-to-sterile segregation ratios of 220:68 and 241:68, respectively. All these genetic data demonstrated that MS9 is a single-gene controlled recessive locus. Compared with Ningchun 4, *ms9* showed no obvious phenotypic differences during the vegetative growth stage. At the flowering stage, however, the spikes of *ms9* were looser with significantly wider-opening glumes; its anthers were thin, shrunken and indehiscent. Pollen viability was determined through iodine-potassium iodide (I₂-KI) staining. Mature pollen of Ningchun 4 appeared as a full, round sphere with deeply stained starch grains, while no visible pollen grains were detected in the anthers of the *ms9* mutant (see Fig. 1).

### Example 2. Map-based cloning of the MS9 gene

Since the *ms9* mutant was generated through EMS mutagenesis, we employed a combined strategy of MutMap analysis and map-based cloning to clone the causal gene MS9. First, samples for genome resequencing were prepared. Genomic DNA from 87 sterile individuals in the F₂ population derived from the cross between *ms9* and Ningchun 4, was pooled as the mutant DNA bulk, and genomic DNA from 72 individuals with the wild-type genotype (determined by F₃ phenotypic analysis) was pooled as the wild-type DNA bulk. Both bulks were subjected to whole-genome resequencing, with 500 G raw data generated each pool. Following MutMap analysis, a large number of single nucleotide polymorphism sites (SNPs) were identified in the mutant, predominantly distributed within the 600-800 Mb interval on the long arm of the wheat chromosome 2A with a distinct peak at 700 Mb position. This result indicates that the causal gene *MS9* is most likely located within this region (Fig. 2).

Based on the MutMap primary mapping results, 5 SNP markers were designed near the 700 Mb region on the chromosome 2A, enabling the initial mapping of the MS9 locus to a 1.37 Mb region within the wheat genome. To achieve fine mapping of the *MS9* locus, additional SNP markers within the range of 1.37 Mb were developed. Using 543 sterile individuals, the target region was narrowed down to a 67.8 Kb region. A total of five protein-coding genes were annotated within this region, and only the second gene harbors a SNP that results in a missense mutation, indicating that it is the most likely candidate for *MS9*, which was designed as *TaMS9* (Fig. 3).

A single nucleotide substitution from G to A is present at the +3541 position relative to the ATG start codon of *TaMS9*. The mutated genomic sequence is shown in SEQ ID NO: 4, and the corresponding mutated CDS sequence is provided in SEQ ID NO: 5. This single nucleotide substitution results in a missense mutation at the 725^{th} residue, where glycine (Gly) is replaced by glutamic acid (Glu). The resulting amino acid sequence is shown in SEQ ID NO: 6.

There is no difference in the *TaMS9* genomic DNA sequence between the wheat varieties Ningchun4 and Chinese Spring at all, with the genomic, CDS, and protein sequence shown in SEQ ID NO: 1, SEQ ID NO: 2, and SEQ ID NO: 3, respectively.

### Example 3. Expression analysis of the TaMS9 gene

According to the gene structure of *TaMS9*, semi-quantitative RT-PCR primers *TaMS9*-RTF and *TaMS9*-RTR (SEQ ID NO: 7 and 8), spanning introns, were designed based on the cDNA sequence. Total RNA was isolated from various tissues of Chinese Spring, including roots, stems, leaves, inflorescences at different developmental stages, lemmas, paleae, pistils and anthers of distinct developmental phases, and was subsequently reverse-transcribed into cDNA. Then the expression pattern of *TaMS9* was analyzed with *ACTIN* gene as an internal control. The results showed that *TaMS9* is ubiquitously expressed in all examined wheat tissues and organs, including anthers at diverse developmental stages (Fig. 4).

The primer sequences used were as follows:
*TaMS9*-RTF: 5^{'}-GTGCTTGTCAAGAATACCGACAAC-3^{'} (SEQ ID NO: 7).
*TaMS9*-RTR: 5^{'}-GATGATGGATGCTAACTGCTCCACT -3^{'} (SEQ ID NO: 8).
ACTIN-RTF: 5^{'}-TCAGCCATACTGTGCCAATC-3^{'} (SEQ ID NO: 9).
ACTIN-RTR: 5^{'}-CTTCATGCTGCTTGGTGC-3^{'} (SEQ ID NO: 10).

### Example 4. Transgenic functional complementation of wheat ms9 mutants

Firstly, a stable transformation vector was constructed for functional complementation of wheat *ms9* mutants. With the binary expression vector P1300-BAR as a backbone, a 9411 bp *TaMS9* genomic sequence (as shown in SEQ ID NO: 11, comprising a 2984 bp promoter, a 4704 bp coding region, and a 1723 bp terminator) was inserted into the vector via In-Fusion method at the *Hind*III and *Sac*I restriction sites, thereby generating the plant expression vector p1300-BAR-*TaMS9*. The promoter sequence is as shown in SEQ ID NO: 12, the genome sequence is as shown in SEQ ID NO: 1, and the terminator sequence is as shown in SEQ ID NO: 13. The plant expression vector was introduced into immature embryos of heterozygous *ms9* mutant plants via *Agrobacterium*-mediated genetic transformation and a total of 23 positive transgenic T₀ plants were obtained. Among them, six T₀ plants were identified as homozygous for the *ms9* mutation using flanking SNP markers. The pollen fertility was assessed by I₂-KI staining, and 4 of the six lines exhibited complete phenotypic rescue.

### Example 5. Functional analysis of the TaMS9 gene promoter

The 2984 bp promoter sequence (SEQ ID NO: 12) of the *TaMS9* gene and the GUS reporter gene (SEQ ID NO: 14) were first inserted into the binary expression vector p1300-BAR via In-Fusion method, generating a p1300-BAR-*TaMS9*::GUS expression vector. The mentioned vector was introduced into wheat immature embryos via *Agrobacterium*-mediated genetic transformation, and 20 transgenic positive plants of the T₀ generation were obtained. The expression pattern of *TaMS9* promoter in different tissues and organs of the transgenic positive T₀ plant was analyzed by GUS staining, and the result was consistent with the semi-quantitative RT-PCR results, indicating that *TaMS9* promoter was ubiquitously expressed.

### Example 6. Application of the TaMS9 gene in Next-Generation Hybrid Breeding Technology in Wheat

A next-generation hybrid breeding technology, developed based on recessive GMS wheat mutants, represents an innovative system for efficient hybrid seed production. The principle was to introduce three closely linked "male fertility restorer gene - pollen inactivation gene - seed color marker gene" into wheat recessive GMS mutants. This generated a maintainer line that functions as both a restorer and a maintainer. Male sterile plants, segregated from self-pollinated progeny of this maintainer line, can be used for hybrid seed production. The present disclosure applies the *ms9* male sterile mutant and the *TaMS9* gene to the next-generation hybrid breeding technology of wheat.

The transgenic vector in the present disclosure was based on the pCAMBIA1300 vector. First, the original resistance gene in pCAMBIA1300 was replaced with a Bar gene driven by the Ubi promoter. Subsequently, the fertility restorer genes *TaMS9*, the maize-derived pollen inactivation gene *ZmBT1-ZmAA* and the blue-grained marker gene were all cloned into the modified vector. The specific information was as follows:
1) a fertility restorer gene expression cassette, wherein the gene promoter, coding region genomic sequence, and terminator are all derived from the wheat cultivar Ningchun 4. The full-length nucleotide sequence is shown in SEQ ID NO: 11, wherein the promoter sequence is shown in SEQ ID NO: 12, the coding region genomic sequence is shown in SEQ ID NO: 1, and the terminator sequence is shown in SEQ ID NO: 13. The amino acid sequence of the protein encoded by this nucleotide sequence is shown in SEQ ID NO: 3.
2) pollen inactivation gene expression cassette *PG47::ZmBT1-ZmAA-IN2-1*, wherein the key functional gene is *ZmAA*, the transit peptide is encoded by *ZmBT1*, and *ZmBT1-ZmAA* (the nucleotide sequence of the *ZmBT1-ZmAA* is shown in SEQ ID NO: 15) is driven by the promoter *PG47* (as shown in SEQ ID NO: 16), and the terminator is *IN2-1* (as shown in SEQ ID NO: 17).
3) the blue-grained marker gene expression cassette is described in patent WO2019090496A1, wherein the expression cassette consists of two tandem genes, *ThMYB1* and *ThR1*. The 3215 bp *ThMYB1* genomic sequence comprises a 1952 bp promoter sequence (shown in SEQ ID NO: 18), a 822 bp genomic sequence (shown in SEQ ID NO: 19), and a 441 bp terminator sequence (shown in SEQ ID NO: 20). The 4422 bp *ThR1* sequence comprises a 2084 bp promoter sequence (shown in SEQ ID NO: 21), a 1720 bp CDS sequence (shown in SEQ ID NO: 22), and a 618 bp terminator sequence (shown in SEQ ID NO: 23).

The vector described above was introduced into immature embryos of *ms9* plants via *Agrobacterium*-mediated genetic transformation *ms9* and 83 transgenic positive plants were obtained. After molecular identification, 21 plants were identified as homozygous *ms9* genotypes. Pollen viability was assessed by I₂-KI staining, and the result showed that approximately 50% of pollens were sterile in 16 out of 21 tested lines. This indicates that the fertility restorer gene expression cassette rescues the male sterility phenotype of *ms9*, and at the same time, the pollen inactivation gene expression cassette inactivates the transgenic half of the total pollen grains. Among the T₁ generation seeds harvested from individual plants, 10 lines exhibited a segregation ratio of 1:1 between normally colored seeds and blue seeds, demonstrating that the blue-grained marker gene expression cassette functions correctly and marks the transgenic seeds as blue-grain. The above results verified that the three expression cassettes in the present disclosure, i.e. the fertility restorer gene, the pollen inactivation gene, and the blue-grained marker gene, function normally, and thus were successfully applied to the next-generation hybrid breeding technology in wheat.

## Claims

1. A fertility-related gene *TaMS9*, wherein a nucleotide sequence of the fertility-related gene *TaMS9* is selected from any one of the sequences in the following group:
(a) the nucleotide sequence as shown in SEQ ID NO: 1 or 2;
(b) a nucleotide sequence encoding the amino acid sequence as shown in SEQ ID NO: 3;
(c) a DNA sequence capable of hybridizing with the DNA sequence described in (a) or (b) under a stringent condition; or
(d) a DNA sequence having at least 80% (preferably at least 85%) sequence similarity to any one of the sequences described in (a) - (c) and possessing a fertility restoration function; or
(e) a DNA sequence complementary to any one of the sequences described in (a) - (d).

2. An expression cassette, an expression vector or an engineered bacterium, wherein the expression cassette, the expression vector or the engineering bacteria comprises the fertility-related gene as claimed in claim 1.

3. Use of a fertility-related gene, an expression cassette, an expression vector, or an engineered bacterium for regulating plant fertility, wherein the fertility-related gene, the expression cassette, the expression vector, or the engineered bacterium comprises any one of the nucleotide sequences shown below:
(a) the nucleotide sequence as shown in SEQ ID NO: 1 or 2;
(b) a nucleotide sequence encoding the amino acid sequence as shown in SEQ ID NO: 3;
(c) a DNA sequence capable of hybridizing with the DNA sequence described in (a) or (b) under a stringent condition; or
(d) a DNA sequence having at least 80% (preferably at least 85%) sequence similarity to any one of the sequences described in (a) - (c) and possessing a fertility restoration function; or
(e) a DNA sequence complementary to any one of the sequences described in (a) - (d).

4. A method for regulating plant fertility, the method comprises: altering the expression level of a fertility-related gene *TaMS9* in a plant through overexpression, suppression, or mutation of the *TaMS9*, thereby regulating plant fertility; wherein the nucleotide sequence of the fertility-related gene *TaMS9* is selected from any one of the sequences in the following group:
(a) the nucleotide sequence as shown in SEQ ID NO: 1 or 2;
(b) a nucleotide sequence encoding the amino acid sequence as shown in SEQ ID NO: 3;
(c) a DNA sequence capable of hybridizing with the DNA sequence described in (a) or (b) under a stringent condition; or
(d) a DNA sequence having at least 80% (preferably at least 85%) sequence similarity to any one of the sequences described in (a) - (c) and possessing a fertility restoration function; or
(e) a DNA sequence complementary to any one of the sequences described in (a) - (d).

5. The method according to claim 4, wherein the mutation comprises substitution, deletion or insertion of one or more nucleotides in the nucleotide sequence of the fertility-related gene.

6. The method according to any one of claims 4-5, wherein the "mutation" comprises, but is not limited to, the following methods, for example, gene mutations caused by a physical or a chemical method, wherein the chemical method comprises mutagenesis caused by the treatment with a mutagen such as EMS, or through a gene silencing method such as RNAi, or through a gene editing method; and a method of gene site-directed mutagenesis comprises, but is not limited to, a gene editing method such as ZFN, TALEN, and/or CRISPR/Cas9.

7. The method according to claim 4, wherein the method comprises complementing the male sterile phenotype caused by the mutation of *TaMS9* gene with the nucleotide sequence of the *TaMS9* gene, thereby restoring the fertility to the *ms9* male sterile line.

8. The method according to claim 7, wherein a sequence of the mutation of *TaMS9* gene is as shown in SEQ ID NO: 4 or 5, and the amino acid sequence is as shown in SEQ ID NO: 6.

9. Use of the method according to any one of claims 4-8 for regulating plant fertility.

10. A method for producing or propagating a male sterile line, the method comprises the following steps:
(a) introducing the following vector into the *ms9* male sterile line, obtaining a maintainer line harboring the following vector, the vector comprises:
a fertility-related gene *TaMS9*, the fertility-related gene *TaMS9* is capable of restores the male fertility of the *ms9* male sterile line; and
a pollen inactivation gene, when expressed in a plant, disturbs the function or formation of a male gamete harboring the pollen inactivation gene, so the fertile male gametes generated in the plant do not harbor the vector; and
a selection marker gene, the selection marker gene is used for sorting transgenic seeds from non-transgenic seeds; and
(b) self-pollinating the maintainer line harboring the vector, whereby both seeds of the *ms9* male sterile line not comprising the vector and seeds of the maintainer line comprising the vector are produced; or pollinating a plant of the *ms9* sterile line with pollen grains of the maintainer line plant, thereby *ms9* propagating seeds of the *ms9* sterile line.

11. The method according to claim 10, wherein the nucleotide sequence of the fertility-related gene *TaMS9* is selected from any one of the sequences in the following group:
(a) the nucleotide sequence as shown in SEQ ID NO: 1 or 2;
(b) a nucleotide sequence encoding the amino acid sequence as shown in SEQ ID NO: 3;
(c) a DNA sequence capable of hybridizing with the DNA sequence described in (a) or (b) under a stringent condition; or
(d) a DNA sequence having at least 80% (preferably at least 85%) sequence similarity to any one of the sequences described in (a) - (c) and possessing a fertility restoration function; or
(e) a DNA sequence complementary to any one of the sequences described in (a) - (d).

12. The method according to claim 11, wherein the fertility-related gene *TaMS9* is driven to express by a pollen-specific promoter; preferably the nucleotide sequence of the pollen-specific promoter is as shown in SEQ ID NO: 12.

13. The method according to any one of claims 10 -12, wherein the pollen inactivation gene comprises but is not limited to the barnase gene, amylase gene, DAM methylase gene, etc.; preferably, the pollen inactivation gene is the maize α-amylase gene, and more preferably, the nucleotide sequence of the maize α-amylase gene is as shown in SEQ ID NO: 15.

14. The method according to claim 13, wherein the pollen inactivation gene is linked to a promoter that is preferentially expressed in male gametes, preferably the promoter is a *PG47* promoter or a *Zm13* promoter.

15. The method according to any one of claims 10-14, wherein the selection marker gene comprises, but is not limited to, a blue-grained gene, an antibiotic resistance gene, an herbicide resistance gene, a fluorescent protein gene, and the like, preferably, the selection marker gene comprises, but is not limited to, a blue-grained gene, a chloramphenicol resistance gene, a hygromycin resistance gene, a streptomycin resistance gene, a spectinomycin resistance gene, a sulfonamide resistance gene, a glyphosate resistance gene, a glufosinate resistance gene, a bar gene, a red fluorescent gene *DsRED*, a *mCherry* gene, a cyan fluorescent protein gene, a yellow fluorescent protein gene, a luciferase gene, a green fluorescent protein gene, and the like.

16. A method for producing or propagating a maintainer line, the method comprises the following steps:
(a) introducing the following vector into the *ms9* male sterile line, obtaining a maintainer line for the *ms9* male sterile line, the vector comprises:
a fertility-related gene *TaMS9*, the fertility-related gene *TaMS9* is capable of restoring the male fertility of the *ms9* male sterile line; and
a pollen inactivation gene, when expressed in a plant, disturbs the function or formation of a male gamete harboring the pollen inactivation gene, so the fertile male gametes generated in the plant do not harbor the vector; and
a selection marker gene, the selection marker gene is used for sorting transgenic seeds from non-transgenic seeds; and
(b) self-pollinating the maintainer line harboring the vector, whereby both seeds of the *ms9* male sterile line not comprising the vector and seeds of the maintainer line comprising the vector are produced.

17. The method according to claim 16, wherein the nucleotide sequence of the fertility-related gene *TaMS9* is selected from any one of the sequences in the following group:
(a) the nucleotide sequence as shown in SEQ ID NO: 1 or 2;
(b) a nucleotide sequence encoding the amino acid sequence as shown in SEQ ID NO: 3;
(c) a DNA sequence capable of hybridizing with the DNA sequence described in (a) or (b) under a stringent condition; or
(d) a DNA sequence having at least 80% (preferably at least 85%) sequence similarity to any one of the sequences described in (a) - (c) and possessing a fertility restoration function; or
(e) a DNA sequence complementary to any one of the sequences described in (a) - (d).

18. The method according to claim 17, wherein the fertility restorer gene *TaMS9* is driven by a pollen-specific promoter, and preferably the nucleotide sequence of the pollen-specific promoter is as shown in SEQ ID NO: 12.

19. The method according to any one of claims 16-18, wherein the pollen inactivation gene comprises, but is not limited to, the barnase gene, amylase gene, DAM methylase, etc.; preferably, the pollen inactivation gene is the maize α-amylase gene, and more; preferably, the nucleotide sequence thereof is as shown in SEQ ID NO: 15.

20. The method according to Claim 19, wherein the pollen inactivation gene is linked to a promoter preferentially-expressed in male gametes, preferably the promoter is a *PG47* promoter or a *Zm13* promoter.

21. The method according to any one of claims 16-20, wherein the selection marker gene comprises, but is not limited to, a blue-grained gene, an antibiotic resistance gene, an herbicide resistance gene, a fluorescent protein gene, and the like, preferably, such selection marker gene comprises, but is not limited to, a blue-grained gene, a chloramphenicol resistance gene, a hygromycin resistance gene, a streptomycin resistance gene, a spectinomycin resistance gene, a sulfonamide resistance gene, a glyphosate resistance gene, a glufosinate resistance gene, a bar gene, a red fluorescent gene *DsRED*, a *mCherry* gene, a cyan fluorescent protein gene, a yellow fluorescent protein gene, a luciferase gene, a green fluorescent protein gene, and the like.
